# EUROPEAN PATENT SPECIFICATION

(11) **EP 1 774 858 B1**
(45) Date of publication and mention of the grant of the patent: **09.05.2018**
(21) Application number: 06122109.9
(22) Date of filing: 11.10.2006
(51) Int. Cl.: A23L 33/175, A61K 9/50, A23P 20/10, A61P 37/00

(54) **Nutritional compositions based on amino acids**
Nährzusammensetzungen auf der Basis von Aminosäuren
Compositions nutritive à base de acide amine

(30) Priority: 13.10.2005 IT FI20050215
(43) Date of publication of application: 18.04.2007
(73) Proprietor: Valpharma International S.P.A., 47864 Pennabilli (IT)
(72) Inventor: Valducci, Roberto, 47039, SAVIGNANO SUL RUBICONE (IT); Avanessian, Serozh, 47827, VILLA VERUCCHIO (IT)
(74) Representative: Gervasi, Gemma

(56) References cited:
- EP-A- 1 110 541
- EP-A- 1 228 763
- WO-A-88/03796
- WO-A-91/04015
- WO-A-2004/028434
- WO-A-2004/095946
- GB-A- 2 038 629
- US-A- 4 146 653
- US-A- 4 753 804
- US-A- 5 393 532

## Description

### FIELD OF THE INVENTION

The present invention relates to the field of nutritional compositions, and in particular to a new nutritional composition for oral administration of amino acids, useful for the treatment of metabolic diseases responding to the administration of selected amino acids, particularly for the treatment of phenylketonuria.

### STATE OF THE ART

Phenylketonuria is an inherited metabolic disease due to the hereditary deficiency of an enzyme, phenylalanine hydroxylase, in affected subjects. This enzyme is responsible for the metabolism of phenylalanine, an essential amino acid common in all protein containing food; therefore the lack of this enzyme results in altered phenylalanine metabolism, giving rise to products that are toxic to the brain.

Moreover, in healthy subjects, not only a correct metabolism of phenylalanine does not result in formation of compounds that are toxic to the brain, but this amino acid is also a neurotransmitter precursor; thus, lack of the phenylalanine hydroxylase enzyme in subjects affected by phenylketonuria results in a double damage of brain function.

Due to the severity of this pathology and the incidence in the population, a mass screening at birth, called "neonatal screening", has been made compulsory for the early diagnosis of phenylketonuria since the '80s. Traditionally, starting from the time when the diagnosis is formulated, therefore since the first days of life, the phenylketonuric subject undergoes a strict aproteic diet, in order to reduce phenylalanine poisoning. This diet involves the administration of amino acid mixtures, obviously devoid of phenylalanine to allow at least a correct muscular and general development.

In fact, until a few year ago, it was believed that a fully developed adolescent could stop such diet but this clinical trend has been now abandoned, due to the most recent findings on the consequences in adults of the non compliance to diet.

In fact, while in infants non-compliance to the above mentioned aproteic diet is detrimental to development, in adolescents and adults it results in neuropsychiatric disorders which are considered of "minor" importance but certainly are not less disruptive to quality of life, including phobias, tremors, concentration difficulties, Intellective Quotient reduction, and similar conditions.

In light of these findings, the current clinical trend, reported in most recent meetings, is to prescribe the aproteic diet treatment throughout life. However, in reality, the strictness of this diet leads phenylketonuric patients to abandon this regimen, generally at the age of 12-14, with the consequences described above. One of the reasons is certainly the inadequacy in terms of taste, smell and flavour of the selected amino acid mixtures that always contain at least one amino acid with unpleasant smell, such as methionine, that is necessary to integrate all proteins and amino acids that a patient cannot acquire with food, due to the restrictions of the aproteic diet.

In fact, in the last few years, many products have been developed that contain amino acid mixtures, and sometimes also vitamins, microelements and mineral salts, generally in the form of granulates to be dissolved in water or other liquids and to be taken in variable amounts, according to body weight and amino acid concentration in the mixture. Not only this powdered products do not solve completely the problem of unpleasantness of certain amino acids, but they also have other practical drawbacks. These relate to the laboriousness of their formulation and to the need for frequent administration, even for school children or adults in the working place or anyway out of home all day. Moreover, it should be said that the high frequency intake of said formulations is due to the fact that currently marketed powdered products do not provide a controlled release of amino acids.

In order to increase patient's compliance, other products have been developed that do not require solubilization of powders. Said products consist of tablets, which represent a formulation that can be conveniently taken also out of home and by young patients. US 5 393 532 discloses coated amino acids with unpleasant taste with bland amino acids used for the treatment of Phenylketronuria. WO 2004/028434 describes a nutritional composition comprising coated amino acids having an immediate release and sustained release profile. However, to mask the unpleasant smell of certain amino acids, said products must contain a large amount of sugars and flavours, thus resulting in tablets that are too large and difficult to swallow if the concentration of amino acids per tablet should be comparable to the usual unitary dose. On the other hand, if the tablet size is reduced, the amino acid concentration for each tablet should be decreased to a point where too many tablets must be administered per day. In fact, to the Applicant's knowledge, there are no products that allow a controlled release of the active principles, even among formulations in capsules and tablets known to date. Therefore, while on the one hand said products increase patient's compliance because of the pleasant smell and taste, on the other hand they can considerably worsen patient's compliance because of other drawbacks not less important than those mentioned above.

Consequently, there is a demand for a nutritional composition based on amino acid mixtures, with an improved patient's compliance, both in terms of improved smell and taste and easy assumptions, hence devoid of the above described drawbacks of known formulations.

### SUMMARY OF THE INVENTION

The Applicant has now found a novel nutritional composition based on mixtures of selected amino acids, in form of pellets. This composition can mask the unpleasant smell of certain amino acids without having to decrease their unitary dose or to increase excessively the amount of sweetening and aromatic excipients in the formulation. Moreover, the present composition can control the release of amino acids, modifying their release and allowing a better utilisation of the mixture.

Therefore, subject of the present invention is a nutritional composition for administration of an amino acid mixture including at least one amino acid with unpleasant taste, in the form of pellets comprising a) an inert core consisting essentially of saccharose and starch, b) a coating comprising said amino acid mixture and c) a polymeric film coating,
wherein said nutritional composition comprises two groups of pellets having different release profiles, the first group having an immediate-release profile and the second having a controlled-release profile.

A process for preparation of the above said nutritional composition is a further subject of the invention.

Further subjects of the invention are a sachet containing a unitary dose of amino acid mixture comprised in the above said composition in the form of pellets, and a kit comprising a multidose container with multiple doses of amino acid mixture comprised in the above said composition in the form of pellets, and a device suitable for quantitative determination of the unitary dose of said amino acid mixture. Features and advantages of the invention will be illustrated in detail in the following description.

### DETAILED DESCRIPTION OF THE INVENTION

The amino acids of possible use in the nutritional compositions of the invention are amino acids in free form or in form of pharmaceutically acceptable salts and, when not otherwise specified, they are amino acids of the L-series.

Preferred amino acids according to the invention are selected, for instance, from the group consisting of L-isoleucine, L-histidine, L-leucine, L-methionine, L-tyrosine, L-tryptophan, L-valine and their mixtures, where L-methionine, L-tryptophan, L-valine and L-isoleucine are amino acids with unpleasant smell and taste. Because they are especially indicated for the treatment of phenylketonuria, the present compositions comprise amino acid mixtures lacking phenylalanine.

According to the invention, the weight ratio between the immediate-release and the controlled-release groups of pellets is preferably comprised between 95:5 and 50:50, while optimal results, both in terms of release and palatability of the composition, were found when the weight ratio between immediate-release and controlled-release groups of pellets is 70:30.

According to a particular embodiment of the invention, both the immediate-release and controlled-release groups of pellets consist of pellets having a coating (b) comprising two layers, the inner layer adjacent to the inert core (a) comprising the amino acids with unpleasant smell and taste, and the inner layer adjacent to the inert core (a) comprising the other amino acids.

According to a further embodiment of the invention, the immediate-release and controlled-release groups of pellets each consists, in turn, of two groups of pellets having a different coating (b), the first group comprising the amino acids with unpleasant smell and taste and the second group comprising the other amino acids.

The coating (b) of the present compositions, in addition to the active principle constituted by the amino acid mixture, typically further comprises a polymeric film coating agent, such as polyvinylpyrrolidone.

The film coating (c) that determines the release profile of the compositions of the invention can comprise, for instance, one or more polymers selected from the group consisting of Shellac, hydroxypropylmethylcellulose (HPMC), polyvinylalcohol (PVA), polyvinylalcohol (PVA) and polyethyleneglycol (PEG) copolymer, polyvinylalcohol (PVA) and "white" polyethyleneglycol (PEG) copolymer, hydroxypropylmethylcellulose (HPMC) and polyvinylalcohol (PVA) copolymer, and their mixtures.

In the pellets having controlled release profile, the coating (c) comprises preferably a polymer selected from the group consisting of Shellac, and its mixtures with hydroxypropylmethylcellulose (HPMC) in which the weight ratio Shellac:HPMC is comprised between 95:5 and 70:30.

Instead, pellets having immediate-release profile comprise preferably a polymer selected from the group consisting of hydroxypropylmethylcellulose (HPMC) and its mixtures with Shellac, in which the weight ratio HPMC: Shellac is comprised between 80:20 and 40:60, polyvinylalcohol (PVA), polyvinylalcohol (PVA) and polyethyleneglycol (PEG) copolymer, polyvinylalcohol (PVA) and "white" polyethyleneglycol (PEG) copolymer, a product marketed under the denomination Opadry® and consisting essentially of hydroxypropylmethylcellulose (HPMC), partially hydrolized polyvinylalcohol (PVA) and polyethyleneglycol (PEG), and HPMC/PVA copolymer.

The nutritional compositions according to the invention may comprise, in addition to the above defined amino acid mixture, one or more active ingredients, vitamins, mineral salts, trace elements, and pharmaceutically acceptable excipients and/or diluents, chosen from those conventionally used in pharmaceutical or nutritional compositions, in order to realise a composition suitable for oral administration.

The compositions of the invention are realized as swallowable pellets, hence easy to carry and to take even when out of home; moreover, such pellets have a small size and a regular shape thus are easy to swallow; in addition, they have a pleasant taste for the patient.

The unitary dose of the present compositions can be, for instance, comprised between 0.5 and 1.5 g of amino acid mixture, and preferably the unitary dose, in amino acids, is 1 g per Kg of patient body weight. The pellets of the invention, in an amount equal to a unitary dose of amino acid mixture, can be used to fill a sachet, and pellets can be administered both by simple swallowing the pellets as such or after their solubilization in water, yoghurt or in other food.

A further subject of the invention is the process for the preparation of the above described pharmaceutical compositions, comprising the following steps:
i) mixing of the amino acids with unpleasant smell and taste, and mixing of the other amino acids, in order to obtain two amino acid mixtures, which are kept separated;
ii) application of the mixture of amino acids with unpleasant smell and taste from step i) on an inert core (a) by nebulization of a polymeric film coating agent, such as polyvinylpirrolidone in alcohol solution, alternating spraying phases with powder application phases;
iii) application of the mixture of amino acids with non-unpleasant smell and taste from step i) on an inert core (a) or on the core already coated with the other amino acid mixture from step ii), by nebulization under the same conditions already mentioned above step ii);
iv) preparation of a solution of a film coating agent in a suitable solvent, and application by nebulization of said solution on the inert core (a) to which the coating (b) from step ii) or step iii) has been applied.

Depending on the desired coating (b), the two amino acid mixtures from step i) are applied on the same inert core at subsequent times, obtaining a coating (b) comprising two layers, the inner layer adjacent to the inert core (a), comprising the amino acids with unpleasant smell and taste, and the outer layer comprising the other amino acids; alternatively the two mixtures are applied separately on two distinct groups of inert cores, in order to realise two groups of pellets with different coating (b), the first group comprising the amino acids with unpleasant smell and taste and the second group comprising the other amino acids.

The following examples are provided to illustrate the invention, but are not intended to limit the scope of the present invention.

### EXAMPLE 1

### Application of amino acids on neutral pellets - Groups A and B separated

The amino acids selected for preparation of the nutritional composition have been divided into the following two groups, based on their organoleptic characteristics:
Group A) composed of the odourless amino acids L-histidine, L-tyrosine and L-leucine;
Group B) composed of the amino acids with unpleasant smell L-methionine, L-tryptophan, L-valine and L-isoleucine.

Individual amino acids, each in amount equal to 2 Kg, have been mixed, while keeping separated the two groups A e B, and the two so obtained mixtures have been separately applied on neutral Sugar Spheres pellets composed of saccharose and starch in a weight ratio 80:20, and having a particle size distribution comprised between 710 and 1000 µm, as follows.

For Group A amino acids, 6 Kg of powdered amino acid mixture have been applied on 760 g of neutral pellets, alternating nebulization of a 10% polyvinylpirrolidone binding solution in ethanol and application of the powder, obtaining spheroids with diameter comprised between 1,680 and 2,000 µm; 3.3 Kg of a 10% polyvinylpirrolidone solution in ethanol have been used to obtain said pellets.

The application has been carried out in a manual coater, using a basket with 25 cm diameter and a 12 rpm rotation, under the following operating conditions:

| | |
|---|---|
| Incoming air temperature | 40-65°C |
| Outgoing air temperature | 30-50°C |
| Product temperature | 30-50°C |
| Nozzle | 0.5 mm |
| Nebulization pressure | 1 bar |

For Group B amino acids, 8 Kg of mixture have been applied on 1.030 Kg of neutral pellets under the conditions already described above for group A amino acids, using 4.200 Kg of a 10% polyvinylpirrolidone solution in ethanol, obtaining spheroids with diameter comprised between 1,680 and 2,000 µm.

### EXAMPLE 2

### Application of amino acids on neutral pellets - Groups A and B together

The amino acids selected for preparation of the nutritional composition have been divided into the following two groups, based on their organoleptic characteristics:
Group A) composed of the odourless amino acids L-histidine, L-tyrosine and L-leucine;
Group B) composed of the amino acids with unpleasant smell L-methionine, L-tryptophan, L-valine and L-isoleucine.

Individual amino acids, each in amount equal to 4.6 Kg, have been mixed, while keeping separated the two groups A and B, and the two so obtained mixtures have been applied on neutral Sugar Spheres pellets composed of saccharose and starch in a weight ratio 80:20 and having a particle size distribution comprised between 710 and 1,000 µm, as follows.

An amount of 18.4 Kg of Group B amino acid mixture has been applied on 4 Kg of neutral pellets by nebulization of a 10% polyvinylpirrolidone solution in ethanol, alternating spraying phases with powder application phases, ultimately obtaining spheroids with diameter comprised between 1,200 and 2,000 µm. The alternating spraying phases and powder application phases have been prolonged until exhaustion of the mixtures.

In view of the organoleptic characteristics of the amino acids in group B, these have been applied before the amino acids of group A.

### EXAMPLE 3

### Film coating process

For each of the three types of spheroids obtained as described above, the spheroids containing either amino acids of Group A or Group B, as in Example 1, and the spheroids with both amino acids of Group A and Group B applied on the same pellet, as in Example 2, two different types of film coating have been realised, one for modified release and the other for immediate release.

### Modified release film coating with Shellac only

An amount of 160 g of a 40% by weight Shellac solution in ethanol, has been applied on 1 Kg of Group A pellets prepared as described above in Example 1 and loaded in a Uniglatt fluid bed equipped with Wurster insert, under the following operating conditions:

| | |
|---|---|
| Incoming air temperature | 40-60°C |
| Outgoing air temperature | 20-40°C |
| Product temperature | 25-35°C |
| Nozzle | 1 mm |
| Nebulization pressure | 1 bar |

Talc is added in small aliquots during nebulization of the solution, for a total amount of 160 g of talc added.

The same type of film coating has been realised also on 1 Kg of Group B pellets, prepared as described above in Example 1, and on 1 Kg of pellets, prepared as described in Example 2, using the same amount of products for the film coating and talc, however performing the application in a manual coater, with a 25 cm diameter basket and 12 rpm rotation, under the following operating conditions:

| | |
|---|---|
| Incoming air temperature | 40-65°C |
| Outgoing air temperature | 30-50°C |
| Product temperature | 30-50°C |
| Nozzle | 0.5 mm |
| Nebulization pressure | 1 bar |

### Modified release film coating with Shellac and hydroxypropylmethylcellulose (HPMC)

An amount of 1.2 Kg of a 40% by weight Shellac solution in ethanol and 300 g of a 5% by weight HPMC solution in ethanol and water (weight ratio ethanol/water = 90/10) have been applied on 9 Kg of pellets, prepared as described above in Example 2, and loaded in a Uniglatt fluid bed equipped with Wurster insert, using the operating conditions reported above.

### Immediate release film coating with hydroxypropylmethylcellulose (HPMC)

An amount of 670 gm of a 5% by weight HPMC solution in ethanol and water (ethanol/water ratio = 90/10) has been applied on 1 Kg of Group A pellets, prepared as described above in Example 1, and on 1 Kg of pellets, prepared as described in Example 2, loaded in a manual coater, with a 25 cm diameter basket and 12 rpm rotation, using the same operating conditions described above for the application in a coater of the modified release film coating.

The same type of film coating has been realised also on 1 Kg of Group B pellets, prepared as described above in Example 1, using the same amounts of products for the film coating, however performing the application in a Uniglatt fluid bed equipped with Wurster insert, using the same operating conditions described above for the application in fluid bed of the modified release film coating.

### Immediate release film coating with polyvinylalcohol (PVA)

An amount of 120 g of a 20% by weight PVA solution in water has been applied on 1 Kg of Group A pellets, prepared as described above in Example 1. The same solution has been applied also on 1 Kg of Group B pellets and on 1 Kg of pellets in Example 2. The application of solutions has been realized by loading the pellets in a Uniglatt fluid bed equipped with Wurster insert, using the same operating conditions described above for the application in fluid bed of the modified release film coating.

### Immediate release film coating with polyvinylalcohol (PVA) and polyethyleneglycol (PEG) copolymer

An amount of 120 g of a 20% by weight aqueous solution of PVA/PEG copolymer has been applied on 1 Kg of Group A pellets, prepared as described above in Example 1. The same solution has been applied also on 1 Kg of Group B pellets and on 1 Kg of pellets as in Example 2. The application of solutions has been realized by loading the pellets in a Uniglatt fluid bed equipped with Wurster insert, using the same operating conditions described above for the application in fluid bed of the modified release film coating.

### Immediate release film coating with polyvinylalcohol (PVA) and "white" polyethyleneglycol (PEG) copolymer

An amount of 120 g of a 20% by weight aqueous solution of PVA/PEG "white" copolymer, composed of PVA/PEG copolymer, polyvinylpirrolidone (PVP) and vinyl acetate (VA) copolymer, Titanium dioxide, kaolin and Sodium lauryl sulphate, have been applied on 1 Kg of Group A pellets, prepared as described above in Example 1. The same solution has been applied also on 1 Kg of Group B pellets and on 1 Kg of pellets as in Example 2. The application of solutions has been realized by loading the pellets in a Uniglatt fluid bed equipped with Wurster insert, using the same operating conditions described above for the application in fluid bed of the modified release film coating.

### Immediate release film coating with Opadry® and HPMC/PVA copolymer

An amount of 120 g of a 20% by weight aqueous solution of HPMC/PVA copolymer and Opadry®, a commercial product whose primary components are HMPC, partially hydrolyzed PVA, PEG, talc and lactose, have been applied on 1 Kg of Group A pellets, prepared as described above in Example 1. The same solution has been applied also on 1 Kg of Group B pellets and on 1 Kg of pellets as in Example 2. The application of solutions has been realized by loading the pellets in a Uniglatt fluid bed equipped with Wurster insert, using the same operating conditions described above for the application in fluid bed of the modified release film coating.

### Immediate release film coating with hydroxypropylmethylcellulose (HPMC) and Shellac

An amount of 4.8 Kg of a 5% by weight HPMC solution in ethanol and water (weight ratio ethanol/water = 90/10) and 300 g of a 40% by weight solution of Shellac in ethanol have been applied on 9 Kg of pellets prepared as described above in Example 2 and loaded in a Uniglatt fluid bed equipped with Wurster insert, using the operating conditions reported above.

## Claims

1. A nutritional composition for oral administration of a mixture of amino acids comprising at least one amino acid with unpleasant taste, in the form of pellets comprising a) an inert core consisting essentially of saccharose and starch, b) a coating comprising said mixture of amino acids and c) a polymeric film coating, said nutritional composition comprising two groups of pellets having different release profiles, the first group having an immediate-release profile and the second having a controlled release-profile.

2. The nutritional composition according to claim 1, wherein the weight ratio between said group of pellets having immediate-release profile and said group of pellets having controlled-release profile is comprised between 95:5 and 50:50.

3. The nutritional composition according to claim 1, wherein the weight ratio between said group of pellets having immediate-release profile and said group of pellets having controlled-release profile is 70:30.

4. The nutritional composition according to claims 1-3, wherein said group of pellets having immediate-release profile and said group of pellets having controlled-release profile both consist of pellets having a coating (b) comprising two layers, the inner layer adjacent to the inert core (a) comprising the amino acids with unpleasant smell and taste and the outer layer comprising the other amino acids.

5. The nutritional composition according to claims 1-3, wherein said group of pellets having immediate-release profile and said group of pellets having controlled-release profile each consists, in turn, of two groups of pellets having a different coating (b), the first group comprising the amino acids with unpleasant smell and taste and the second group comprising the other amino acids.

6. The nutritional composition according to claim 1, wherein said amino acids are selected from the group consisting of L-isoleucine, L-histidine, L-leucine, L-methionine, L-tyrosine, L-tryptophan, L-valine and mixtures thereof, where L-methionine, L-tryptophan, L-valine and L-isoleucine are amino acids with unpleasant smell and taste.

7. The nutritional composition according to claim 1, wherein said mixture of amino acids is constituted by L-isoleucine, L-histidine, L-leucine, L-methionine, L-tyrosine, L-tryptophan and L-valine, where L-methionine, L-tryptophan, L-valine and L-isoleucine are said amino acids with unpleasant smell and taste, said mixture being devoid of phenylalanine.

8. The nutritional composition according to claim 1, wherein said inert core (a) consists of inert pellets composed of saccharose and starch, in a weight ratio 80:20, having a particle size distribution comprised between 710 and 1,000 µm.

9. The nutritional composition according to claim 1, wherein said coating (b) further comprises a polymeric film coating agent.

10. The nutritional composition according to claim 9, wherein said film coating agent is polyvinylpirrolidone.

11. The nutritional composition according to claim 1, wherein said coating (c) comprises a polymer selected from the group consisting of Shellac, hydroxypropylmethylcellulose (HPMC), polyvinylalcohol (PVA), polyvinylalcohol (PVA) and polyethyleneglycol (PEG) copolymer, polyvinylalcohol (PVA) and "white" polyethyleneglycol (PEG) copolymer, hydroxypropylmethylcellulose (HPMC) and polyvinylalcohol (PVA) copolymer, and mixtures thereof.

12. The nutritional composition according to claim 1, wherein said coating (c) in the pellets having controlled release profile, comprises a polymer selected from the group consisting of Shellac, and its mixtures with hydroxypropylmethylcellulose (HPMC) in which the weight ratio Shellac:HPMC is comprised between 95:5 and 70:30.

13. The nutritional composition according to claim 1, wherein said group of pellets having immediate-release profile comprises a polymer selected from the group consisting of hydroxypropylmethylcellulose (HPMC) and its mixtures with Shellac, wherein the weight ratio HPMC: Shellac is comprised between 80:20 and 40:60, polyvinylalcohol (PVA), polyvinylalcohol (PVA) and polyethyleneglycol (PEG) copolymer, polyvinylalcohol (PVA) and "white" polyethyleneglycol (PEG) copolymer, mixture of hydroxypropylmethylcellulose (HPMC) and partially hydrolized polyvinylalcohol (PVA) and hydroxypropylmethylcellulose (HPMC) and polyethyleneglycol (PEG), and polyvinylalcohol (PVA) copolymer.

14. The nutritional composition according to claim 1, further comprising one or more additional active principles.

15. The nutritional composition according to claim 1, further comprising vitamins, mineral salts, and/or trace elements.

16. The nutritional composition according to claim 1, further comprising pharmaceutically acceptable excipients and/or diluents.

17. A process for preparation of the nutritional composition as defined in claims 1-16, comprising the following steps:
i) mixing of the amino acids with unpleasant smell and taste, and mixing of the other amino acids, in order to obtain two amino acid mixtures, which are kept separated;
ii) application of the mixture of amino acids with unpleasant smell and taste from step i) on an inert core (a) by nebulization of a polymeric film coating agent, alternating spraying phases with powder application phases;
iii) application of the mixture of amino acids with non-unpleasant smell and taste from step i) on an inert core (a) or on the core already coated with the other amino acid mixture from step ii), by nebulization of a polymeric film coating agent under the same conditions already mentioned above in step ii);
iv) preparation of a solution of film coating agent in a suitable solvent, and application by nebulization of said solution on the inert core (a) to which the coating (b) as in step ii) or step iii) has been applied.

18. The process according to claim 17, wherein the two mixtures from step i) are applied on the same inert core in subsequent times, in order to realize a coating (b) comprising two layers, the inner layer adjacent to the inert core (a), comprising the amino acids with unpleasant smell and taste, and the outer layer comprising the other amino acids.

19. The process according to claim 17, wherein the two mixtures from step i) are applied separately on two different groups of inert cores, in order to realise two groups of pellets with different coating (b), the first group comprising the amino acids with unpleasant smell and taste and the second group comprising the other amino acids.

20. The process according to claim 17, wherein said polymeric film coating agent of step ii) or iii) is polyvinylpirrolidone in alcoholic solution.

21. A sachet containing a unitary dose of amino acid mixture comprised in the composition in the form of pellets as defined in claims 1-16.

22. A kit comprising a multidose container containing multiple doses of amino acid mixture comprised in the composition in the form of pellets, as defined in claims 1-16, and a device suitable for quantitative determination of the unitary dose of said amino acid mixture.

## Patentansprüche

1. Nährstoffzusammensetzung zur oralen Verabreichung einer Mischung von Aminosäuren mit zumindest einer Aminosäure mit unangenehmem Geschmack in Form von Tabletten, die
a) einen neutralen Kern, der im Wesentlichen aus Saccharose und Speisestärke besteht,
b) eine Beschichtung mit der Mischung von Aminosäuren und
c) eine Polymerfilm-Beschichtung aufweisen,
wobei die Nährstoffzusammensetzung zwei Gruppen von Tabletten mit unterschiedlichem Freisetzungsprofil aufweist, und wobei die erste Gruppe ein Profil der sofortigen Freisetzung und die zweite Gruppe ein Profil der kontrollierten Freisetzung aufweist.

2. Nährstoffzusammensetzung gemäß Anspruch 1, wobei ein Gewichtsverhältnis zwischen der Gruppe der Tabletten mit dem Profil der sofortigen Freisetzung und der Gruppe der Tabletten mit dem Profil der kontrollierten Freisetzung zwischen 95 : 5 und 50 : 50 vorgesehen ist.

3. Nährstoffzusammensetzung gemäß Anspruch 1, wobei ein Gewichtsverhältnis zwischen der Gruppe der Tabletten mit dem Profil der sofortigen Freisetzung und der Gruppe der Tabletten mit dem Profil der kontrollierten Freisetzung von 70 : 30 vorgesehen ist.

4. Nährstoffzusammensetzung gemäß den Ansprüchen 1 bis 3, wobei sowohl die Gruppe der Tabletten mit dem Profil der sofortigen Freisetzung als auch die Gruppe der Tabletten mit dem Profil der kontrollierten Freisetzung, die beide Tabletten aufweisen, die eine Beschichtung (b) mit zwei Schichten aufweisen, wobei die innere Schicht, benachbart zum neutralen Kern (a) die Aminosäuren mit dem unangenehmen Geruch und Geschmack aufweist und die äußere Schicht die anderen Aminosäuren aufweist.

5. Nährstoffzusammensetzung gemäß den Ansprüchen 1 bis 3, wobei die Gruppe der Tabletten mit dem Profil der sofortigen Freisetzung und die Gruppe der Tabletten mit dem Profil der kontrollierten Freisetzung wiederum zwei Gruppen von Tabletten mit unterschiedlicher Beschichtung (b) aufweisen, wobei die erste Gruppe die Aminosäuren mit dem unangenehmen Geruch und Geschmack aufweist und die zweite Gruppe die anderen Aminosäuren aufweist.

6. Nährstoffzusammensetzung gemäß Anspruch 1, wobei die Aminosäuren ausgewählt sind aus der Gruppe von L-Isoleucin, L-Histidin, L-Leucin, L-Methionin, L-Tyrosin, L-Tryptophan, L-Valin, und Mischungen daraus, wobei L-Methionin, L-Tryptophan, L-Valin und L-Isoleucin die Aminosäuren mit dem unangenehmen Geruch und Geschmack sind.

7. Nährstoffzusammensetzung gemäß Anspruch 1, wobei die Mischung von Aminosäuren gebildet wird aus L-Isoleucin, L-Histidin, L-Leucin, L-Methionin, L-Tyrosin, L-Tryptophan und L-Valin, wobei L-Methionin, L-Tryptophan, L-Valin und L-Isoleucin die Aminosäuren mit dem unangenehmen Geruch und Geschmack sind und die Mischung frei von Phenylalanin ist.

8. Nährstoffzusammensetzung gemäß Anspruch 1, wobei der neutrale Kern (a) aus neutralen Tabletten besteht, die aus Saccharose und Speisestärke im Gewichtsverhältnis von 80 : 20 zusammengesetzt sind und eine Partikelgrößenvariation zwischen 710 und 1.000 µm aufweisen.

9. Nährstoffzusammensetzung gemäß Anspruch 1, wobei die Beschichtung (b) des Weiteren ein Polymerfilm-Beschichtungsmittel aufweist.

10. Nährstoffzusammensetzung gemäß Anspruch 9, wobei das Polymerfilm-Beschichtungsmittel Polyvinyl-Pirrolidon ist.

11. Nährstoffzusammensetzung gemäß Anspruch 1, wobei die Beschichtung (c) ein Polymer aufweist, das ausgewählt ist aus der Gruppe von Schellack, Hydroxypropylmethylcellulose (HPMC), Polyvinyl-Alkohol (PVA), Polyvinyl-Alkohol (PVA) und Polyethylenglykol (PEG) - Copolymer, Polyvinyl-Alkohol (PVA) und "weisses" Polyethylenglykol (PEG) - Copolymer, Hydroxypropylmethylcellulose (HPMC) und Polyvinyl-Alkohol (PVA) - Copolymer und Mischungen davon.

12. Nährstoffzusammensetzung gemäß Anspruch 1, wobei die Beschichtung (c) in den Tabletten mit dem Profil der kontrollierten Freisetzung ein Polymer aufweist, das ausgewählt ist aus der Gruppe bestehend aus Schellack und seinen Mischungen mit Hydroxypropylmethylcellulose (HPMC), einem Gewichtsverhältnis Schellack : HPMC zwischen 95 : 5 und 70 : 30.

13. Nährstoffzusammensetzung gemäß Anspruch 1, wobei die Gruppe der Tabletten mit dem Profil der sofortigen Freisetzung ein Polymer aufweist, das ausgewählt ist aus der Gruppe von Hydroxypropylmethylcellulose (HPMC) und seinen Mischungen mit Schellack, einem Gewichtsverhältnis HPMC : Schellack zwischen 80 : 20 und 40 : 60, Polyvinyl-Alkohol (PVA), Polyvinyl-Alkohol (PVA) und Polyethylenglykol (PEG) - Copolymer, Polyvinyl-Alkohol (PVA) und "weisses" Polyethylenglykol (PEG) - Copolymer, Mischung aus Hydroxypropylmethylcellulose (HPMC) und partiell hydrolisiertem Polyvinyl-Alkohol (PVA) und Hydroxypropylmethylcellulose (HPMC) und Polyethylenglykol (PEG) und Polyvinyl-Alkohol (PVA) - Copolymer.

14. Nährstoffzusammensetzung gemäß Anspruch 1, des Weiteren einen oder mehrere zusätzliche Wirkstoffe aufweisend.

15. Nährstoffzusammensetzung gemäß Anspruch 1, weiter aufweisend Vitamine, Mineralsalze und / oder Spurenelemente.

16. Nährstoffzusammensetzung gemäß Anspruch 1, weiter aufweisend pharmazeutisch geeignete Hilfsstoffe und / oder Verdünnungsmittel.

17. Verfahren zur Herstellung einer Nährstoffzusammensetzung gemäß den Ansprüchen 1 bis 16, aufweisend die folgenden Schritte:
i) Mischen der Aminosäuren mit unangenehmem Geruch und Geschmack und Mischen der anderen Aminosäuren, um zwei Mischungen von Aminosäuren zu erhalten, die voneinander getrennt bleiben;
ii) Auftragen der Mischung der Aminosäuren mit unangenehmem Geruch und Geschmack aus Schritt i) auf einen neutralen Kern (a) durch Zerstäubung eines Polymerfilm-Beschichtungsmittels im Wechsel von Sprühphasen mit Pulverauftragsphasen;
iii) Auftragen der Mischung der Aminosäuren mit nicht unangenehmem Geruch und Geschmack aus Schritt i) auf einen neutralen Kern (a) oder auf den Kern, der bereits mit der anderen Mischung der Aminosäuren aus Schritt ii) beschichtet ist, durch Zerstäubung eines Polymerfilm-Beschichtungsmittels unter denselben Bedingungen, wie bereits in Schritt ii) erwähnt;
iv) Zubereitung einer Lösung von Film-Beschichtungsmittel in einem geeigneten Lösungsmittel und Auftragen der Lösung auf den neutralen Kern (a), auf den bereits die Beschichtung (b) aus Schritt ii) oder Schritt iii) aufgetragen wurde.

18. Verfahren gemäß Anspruch 17, wobei die beiden Mischungen aus Schritt i) nacheinander auf denselben neutralen Kern aufgetragen werden, um eine Beschichtung (b) zu erhalten, die zwei Schichten aufweist, wobei die innere Schicht, benachbart zum neutralen Kern (a), die Aminosäuren mit dem unangenehmen Geruch und Geschmack aufweist und die äußere Schicht die anderen Aminosäuren aufweist.

19. Verfahren gemäß Anspruch 17, wobei die beiden Mischungen aus Schritt i) getrennt voneinander auf zwei unterschiedliche Gruppen von neutralen Kernen aufgetragen werden, um zwei Gruppen von Tabletten mit unterschiedlicher Beschichtung (b) zu erhalten, wobei die erste Gruppe die Aminosäuren mit dem unangenehmen Geruch und Geschmack aufweist und die zweite Gruppe die anderen Aminosäuren aufweist.

20. Verfahren gemäß Anspruch 17, wobei das Polymerfilm-Beschichtungsmittel aus Schritt ii) oder iii) Polyvinyl-Pirrolidon in Alkohollösung ist.

21. Portionsbeutel mit einer unitären Dosis einer Mischung von Aminosäuren in der Zusammensetzung in Form von Tabletten gemäß den Ansprüchen 1 bis 16.

22. Set, aufweisend einen Mehrfachdosis-Behälter, der mehrere Dosen einer Mischung von Aminosäuren in der Zusammensetzung in Form von Tabletten gemäß den Ansprüchen 1 bis 16 beinhaltet, sowie eine Vorrichtung, die die quantitative Bestimmung der unitären Dosis von Aminosäuren-Mischungen ermöglicht.

## Revendications

1. Composition nutritionnelle pour une administration orale d'un mélange d'acides aminés comprenant au moins un acide aminé de goût désagréable, sous la forme de granules comprenant a) un noyau inerte consistant essentiellement en du saccharose et de l'amidon, b) un enrobage comprenant ledit mélange d'acides aminés et c) un pelliculage polymérique, ladite composition nutritionnelle comprenant deux groupes de granules ayant des profils de libération différents, le premier groupe ayant un profil de libération immédiate et le second ayant un profil de libération contrôlée.

2. Composition nutritionnelle selon la revendication 1, dans laquelle le rapport en poids entre ledit groupe de granules ayant un profil de libération immédiate et ledit groupe de granules ayant un profil de libération contrôlée est compris entre 95:5 et 50:50.

3. Composition nutritionnelle selon la revendication 1, dans laquelle le rapport en poids entre ledit groupe de granules ayant un profil de libération immédiate et ledit groupe de granules ayant un profil de libération contrôlée est de 70:30.

4. Composition nutritionnelle selon les revendications 1 à 3, dans laquelle ledit groupe de granules ayant un profil de libération immédiate et ledit groupe de granules ayant un profil de libération contrôlée consistent tous deux en des granules ayant un enrobage (b) comprenant deux couches, la couche interne étant adjacente au noyau inerte (a) comprenant les acides aminés d'odeur et de goût désagréables et la couche externe comprenant les autres acides aminés.

5. Composition nutritionnelle selon les revendications 1 à 3, dans laquelle ledit groupe de granules ayant un profil de libération immédiate et ledit groupe de granules ayant un profil de libération contrôlée consistent chacun, tour à tour, en deux groupes de granules ayant un enrobage différent (b), le premier groupe comprenant les acides aminés d'odeur et de goût désagréables et le second groupe comprenant les autres acides aminés.

6. Composition nutritionnelle selon la revendication 1, dans laquelle lesdits acides aminés sont choisis dans le groupe consistant en la L-isoleucine, la L-histidine, la L-leucine, la L-méthionine, la L-tyrosine, le L-tryptophane, la L-valine, et leurs mélanges, où la L-méthionine, le L-tryptophane, la L-valine et la L-isoleucine sont des acides aminés d'odeur et de goût désagréables.

7. Composition nutritionnelle selon la revendication 1, dans laquelle ledit mélange d'acides aminés est constitué par la L-isoleucine, la L-histidine, la L-leucine, la L-méthionine, la L-tyrosine, le L-tryptophane et la L-valine, où la L-méthionine, le L-tryptophane, la L-valine et la L-isoleucine sont lesdits acides aminés d'odeur et de goût désagréables, ledit mélange étant exempt de phénylalanine.

8. Composition nutritionnelle selon la revendication 1, dans laquelle ledit noyau inerte (a) consiste en des granules inertes composés de saccharose et d'amidon, dans un rapport en poids de 80:20, ayant une distribution de taille de particule comprise entre 710 et 1 000 µm.

9. Composition nutritionnelle selon la revendication 1, dans laquelle ledit enrobage (b) comprend en outre un agent de pelliculage polymérique.

10. Composition nutritionnelle selon la revendication 9, dans laquelle ledit agent de pelliculage est la poly(vinylpyrrolidone).

11. Composition nutritionnelle selon la revendication 1, dans laquelle ledit enrobage (c) comprend un polymère choisi dans le groupe consistant en la gomme-laque, l'hydroxypropylméthylcellulose (HPMC), le poly(alcool vinylique) (PVA), un copolymère de poly(alcool vinylique) (PVA) et de poly(éthylèneglycol) (PEG), un copolymère de poly(alcool vinylique) (PVA) et de poly(éthylèneglycol) (PEG) « blanc », un copolymère d'hydroxypropylméthylcellulose (HPMC) et de poly(alcool vinylique) (PVA), et leurs mélanges.

12. Composition nutritionnelle selon la revendication 1, dans laquelle ledit enrobage (c) dans les granules ayant un profil de libération contrôlée, comprend un polymère choisi dans le groupe consistant en la gomme-laque, et ses mélanges avec l'hydroxypropylméthylcellulose (HPMC) dans laquelle le rapport en poids gomme-laque:HPMC est compris entre 95:5 et 70:30.

13. Composition nutritionnelle selon la revendication 1, dans laquelle ledit groupe de granules ayant un profil de libération immédiate comprend un polymère choisi dans le groupe consistant en l'hydroxypropylméthylcellulose (HPMC) et ses mélanges avec la gomme-laque, dans laquelle le rapport en poids HPMC:gomme-laque est compris entre 80:20 et 40:60, le poly(alcool vinylique) (PVA), un copolymère de poly(alcool vinylique) (PVA) et de poly(éthylèneglycol) (PEG), un copolymère de poly(alcool vinylique) (PVA) et de poly(éthylèneglycol) (PEG) « blanc », un mélange d'hydroxypropylméthylcellulose (HPMC) et de poly(alcool vinylique) (PVA) partiellement hydrolysé et un copolymère d'hydroxypropylméthylcellulose (HPMC) et de poly(éthylèneglycol) (PEG), et de poly(alcool vinylique) (PVA).

14. Composition nutritionnelle selon la revendication 1, comprenant en outre un ou plusieurs principes actifs additionnels.

15. Composition nutritionnelle selon la revendication 1, comprenant en outre des vitamines, des sels minéraux, et/ou des oligoéléments.

16. Composition nutritionnelle selon la revendication 1, comprenant en outre des excipients et/ou diluants pharmaceutiquement acceptables.

17. Procédé de préparation de la composition nutritionnelle telle que définie dans l'une quelconque des revendications 1 à 16, comprenant les étapes suivantes :
i) mélange des acides aminés d'odeur et de goût désagréables, et mélange des autres acides aminés, afin d'obtenir deux mélanges d'acides aminés, qui sont maintenus séparés ;
ii) application du mélange d'acides aminés d'odeur et de goût désagréables de l'étape i) sur un noyau inerte (a) par nébulisation d'un agent de pelliculage polymérique, en alternant des phases de pulvérisation avec des phases d'application de poudre ;
iii) application du mélange d'acides aminés d'odeur et de goût non désagréables de l'étape i) sur un noyau inerte (a) ou sur le noyau déjà enrobé du mélange d'autres acides aminés issus de l'étape ii), par nébulisation d'un agent de pelliculage polymérique dans les mêmes conditions que celles déjà mentionnées ci-dessus dans l'étape ii) ;
iv) préparation d'une solution d'un agent de pelliculage dans un solvant adéquat, et application par nébulisation de ladite solution sur le noyau inerte (a) auquel a été appliqué l'enrobage (b) comme dans l'étape ii) ou l'étape iii).

18. Procédé selon la revendication 17, dans lequel les deux mélanges issus de l'étape i) sont appliqués sur le même noyau inerte à des temps consécutifs, afin de réaliser un enrobage (b) comprenant deux couches, la couche interne étant adjacente au noyau inerte (a), comprenant les acides aminés d'odeur et de goût désagréables, et la couche externe comprenant les autres acides aminés.

19. Procédé selon la revendication 17, dans lequel les deux mélanges de l'étape i) sont appliqués séparément sur deux groupes différents de noyaux inertes, afin de réaliser deux groupes de granules d'enrobage (b) différent, le premier groupe comprenant les acides aminés d'odeur et de goût désagréables et le second groupe comprenant les autres acides aminés.

20. Procédé selon la revendication 17, dans lequel ledit agent de pelliculage polymérique de l'étape ii) ou iii) est la poly(vinylpyrrolidone) en solution alcoolique.

21. Sachet comprenant une dose unitaire de mélange d'acides aminés compris dans la composition sous la forme de granules telle que définie dans les revendications 1 à 16.

22. Trousse comprenant un contenant multidose contenant de multiples doses de mélange d'acides aminés compris dans la composition sous la forme de granules, telle que définie dans les revendications 1 à 16, et un dispositif convenant pour déterminer quantitativement la dose unitaire dudit mélange d'acides aminés.
